(19) **Europäisches Patentamt**
**European Patent Office**
**Office européen des brevets**

(11) **EP 1 636 595 B1**

(12) **EUROPEAN PATENT SPECIFICATION**

(45) Date of publication and mention
of the grant of the patent:
**29.10.2008 Bulletin 2008/44**

(51) Int Cl.:
*G01N 33/86* (2006.01)    *G01N 33/49* (2006.01)

(21) Application number: **04736891.5**

(22) Date of filing: **15.06.2004**

(86) International application number:
**PCT/SE2004/000940**

(87) International publication number:
**WO 2004/111656 (23.12.2004 Gazette 2004/52)**

(54) **COAGULATION TESTS AT AMBIENT TEMPERATURE**

KOAGULATIONSTEST BEI UMGEBUNGSTEMPERATUR

TEST DE COAGULATION A TEMPERATURE AMBIANTE

(84) Designated Contracting States:
**AT BE BG CH CY CZ DE DK EE ES FI FR GB GR HU IE IT LI LU MC NL PL PT RO SE SI SK TR**

(30) Priority: **15.06.2003 SE 0301785**

(43) Date of publication of application:
**22.03.2006 Bulletin 2006/12**

(73) Proprietor: **Zafena AB**
**S-59076 Vreta Kloster (SE)**

(72) Inventor: **RANBY, Mats**
**S-590 76 Vreta Kloster (SE)**

(74) Representative: **Nilsson, Brita Linnea et al**
**Zacco Sweden AB**
**P.O. Box 23101**
**104 35 Stockholm (SE)**

(56) References cited:
**EP-A1- 0 476 923**          **WO-A1-93/22453**
**US-A- 3 951 606**          **US-B1- 6 338 821**

- **ALLINGTON M.J.: 'Owren's method for the control of anticoagulant therapy.' JOURNAL OF CLINICAL PATHOLOGY vol. 11, no. 1, January 1958, pages 62 - 68**

- **DREHER A. ET AL: 'Temperaturabhängigkeit der Blutgerinnung in vitro' BLUT vol. 36, 1978, SPRINGER VERLAG, pages 231 - 238, XP008078793**
- **OWREN P.A.; AAS K.: 'The control of dicumarol therapy and the quantitative determination of prothrombin and proconvertin.' SCANDINAVIAN JOURNAL OF CLINICAL AND LABORATORY INVESTIGATION vol. 3, no. 3, 1951, pages 201 - 208**
- **ABDIMAJID OSMAN: "Linköping university medical dissertations:1000" 2007, pages 44-46, Sweden ISSN: 0345-0082 ISBN: 978 91 85715 45 9**
- **OSMAN ABDIMAJID ET AL: 'Plasma sample dilution improves the correlation between reagents for PT methods' BLOOD COAGULATION & FIBRINOLYSIS vol. 18, no. 3, 2007, ENGLAND, pages 1 - 7**
- **DATABASE MEDLINE [Online] US NATIONAL LIBRARY OF MEDICINE (NLM), BETHESDA, MD, US 1983 LAHARRAGUE P. ET AL: 'Evaluation of Owren's thrombotest in the monitoring of treatment with antivitamin K.'**

Remarks:
The file contains technical information submitted after the application was filed and not included in this specification

## Description

The present invention relates to a method of determining prothrombin time (PT) in a whole blood, anti-coagulated blood, blood plasma or anti-coagulated blood plasma sample at ambient temperature.

## Background of the invention

Prothrombin time (PT) is a laboratory diagnostic coagulation test by which the functionality of the extrinsic coagulation pathway is assessed. Coagulation tests are defined as test performed on biological fluids such as blood, anti-coagulated blood, blood plasma or anti-coagulated blood plasma, for which tests the end-point is a coagulation phenomena, such as formation of a gel or a clot or the dissolution of gel or clot. In more modem terminology, the end-point of a coagulation test involves the formation of fibrin from fibrinogen or the dissolution of fibrin into fibrin degradation products.

Prothrombin time (PT) tests are used to diagnose liver disease and coagulation anomalies of the blood. PT tests are also used to monitor antithrombotic therapy by vitamin K antagonists. In performing a PT test, sample and liquid thromboplastin reagent are mixed at 37°C and the time needed for the mixture to clot is determined. When the test was first devised more than fifty years ago, this clotting time was identical to the PT, but today, to accommodate variations in test procedure properties, the PT is expressed in International Normalized Ratio (INR), see below.

PT tests measure the time from exposure to membrane bound tissue factor (thromboplastin) to the detection of a clot. Two types of PT tests are recognized, Quick PT and Owren PT, named after the first author as they were first described in the literature, Quick A et al. The prothrombin time in hemophilia and in obstructive jaundice. J Biol Chem 1935; 109: 73-4 and Owren P and Aas K. The control of dicumarol therapy and quantitative determination of prothrombin and proconvertin. Scand J Clin Lab Invest 1951; 1951; 201-8. Both the Owren PT and the Quick PT are wet-chemistry coagulation tests, i.e. the sample is mixed with liquid (not dry) reagent. The difference in the procedures resides in the fact that the Quick PT reagent contains only thromboplastin whereas the Owren PT reagent also contains fibrinogen and coagulation factor V. The fibrinogen content of the reagent may be viewed as discriminatory. If the PT reagent contains sufficient amounts of fibrinogen to increase the fibrinogen content of the mixture of sample and reagent by at least 0.1 g/L then it is an Owren-type PT test, not a Quick PT. This is even more true when the increase is at least 0.5 g/L. Another cardinal distinguishing feature is that the relationship between reagent and sample is greater than four, typically greater than ten; in a Owren-type PT procedure whereas it is hardly ever greater than two in the Quick-type. The publication Lindahl et al. Plasma-based calibration of Owren-type prothrombin time with results expressed in INR. Thromb Haemost 2004; 91: 1223-31, submitted to publication to several journals during the past six years, was published 8 months after the filing of the priority application of the present invention, but may be viewed as containing relevant background data.

Today, the result of a PT test is not expressed in clotting time (CT) but rather in International Normalized Ratio (INR). The INR is obtained by comparing the CT for the sample with a sample of an average normal individual, normal clotting time ((NCT). The INR is the ratio between CT and NCT raised to the power of ISI, the International Sensitivity Index. See the above given references of van den Besselaar et al and Pollar et al. The NCT and ISI are properties of the certain PT test procedure and are obtained by calibrating the test. In the calibration of a given PT test procedure the results are directly or indirectly aligned with those of a reference PT test procedure.

According to prior art, laboratory diagnostic coagulation tests, such as PT, are performed at 37°C to mimic the physiological situation. The difficulties in standardizing a PT test at this temperature, or at any temperature, are considerable as is evident from on-going international work in the area; van den Besselaar AM. International normalized ratio: towards improved accuracy. Thromb Haemost 1999; 82: 1562-3, and Pollar L et al Concerted Action on Anticoagulation (ECAA) - the multicentre calibration of rabbit and human ECAA reference thromboplastins. Steering Committee. Thromb Haemost 1996; 76: 977-82. However, the lock-in of PT at 37°C presents a hurdle in design of test procedures, especially for those intended to be used close to the patient, i.e. in point of care (POC) PT testing. To be able to maintain a temperature of 37°C during the test performance, a thermostat set at 37°C is needed, and a temperature-equilibration time must be allowed. The need of a thermostat adds much hardware and costs to the currently available instrumentation and hampers the development toward increased point of care (POC) testing. The notion to perform a PT test at ambient temperature, especially at room temperature, is very attractive.

Many PT tests are performed on blood plasma at highly automated centralized laboratories. Although this may be economical for generating the test results, it may not be overall economical from a patient-care point of view. The reason is the time required to transport samples to the laboratory and to get the results back. This time can be hours to days. This is long time compared to the 15 minutes or less that would be required for a PT test to be performed next to the patient, that is at the point of care (POC). The transportation time can delay the start of optimal treatment and can force the patient to make additional visits to the physician. In addition, a centralized PT analysis will require a vein puncture, a relatively large blood sample volume and a sturdy blood container. A POC PT analysis only requires a fingerstick, a small blood volume and no sample container for transportation of the sample. Considerations of this kind have prompted the development of POC PT test procedures intended to be performed at doctors' offices, at small laboratories and at patients' homes.

As alluded to above, the PT test represents a considerable standardization challenge which calls for caution in moving away from established test conditions. A tempting move in the design of POC PT test procedures is the move from established wet chemistry to dry chemistry because the latter is more readily adapted to the demands of a POC laboratory test situation. Inventive aspects of dry chemistry POC PT test design are found in US patent 6,402,704B1 to McMorrow, US patent 6,103,196 to Yassinzadeh et al, US patent 5,302,348 to Cusack et al, US patent 4,849,340 to Oberhardt, US patent 3,951,606 to Moyer et al, US patent 6,338,821 to Jina, and the International patent application WO 93/22453 of Zweig. The latter of these describes a test article for determining coagulation capability in a blood sample comprising a porous membrane having coagulation initiator and substrate impregnated therein. The test is <u>not</u> a coagulation test since the end-point is <u>not</u> a detected clotting, what is detected is the cleavage of a synthetic substrate by some enzyme, possibly thrombin. In the description of the invention, it is proposed that the test response could, by mathematical means, be made equivalent to the PT of the sample also when the test is performed at ambient room temperature. No quantitative proof of this is presented.

There are about 4 million European patients on vitamin K antagonist therapy who need to have their PT determined about 20 times per year. This makes for 80 million PT tests per year, each of which requires about 15 minutes of health care time and contributes with about 10 Euro per test in health care costs, not counting test materials which add about 0.5 Euro per test. The health care cost of PT testing in monitoring antithrombotic therapy, not counting test materials, is thus about 800 million Euro. If these health care costs could be reduced by 20% by increased frequency of home POC PT testing, while enhance the quality of life for the patient, health care savings of 160 million Euro would result. Even after an offset by about 2 Euro per test in increased material costs, the savings in the health care sector would be about 130 million Euro.

**Description of the invention**

The present invention is directed to a method of determ ining , in a coagulation test, prothrombin time (PT) in a whole blood, anti-coagulated blood, blood plasma or anti-coagulated blood plasma sample. The method comprises the steps of

a) adding a defined volume of the sample to a defined volumes of a liquid reagent comprising thromboplastin and fibrinogen in a ratio between the defined volumes of the reagent and the sample of at least four, the amount of fibrinogen increasing the concentration of fibrinogen in the mixture by at least 0.1 g/L, or vice versa, in a vessel,

b) measuring the temperature of the reaction mixture of a) having an ambient temperature in the range of 18°C to 35°C, the steps a) and b) being performed in an optional order, followed by

c) determining a clotting time (CT) by registering the time from the addition in a) until a clot formation is detected in the vessel,

and

d) calculating the prothrombin time (PT) based on the temperature of b) and the clotting time (CT) of c), expressed as international Normalized Ratio (INR), by using the equation

$$INR = (CT/(NCT(t))^{ISI(t)}$$

wherein

CT= the clotting time of c) in claim 1,

t = the temperature of b) in claim 1,

NCT(t) = Normal Clotting Time (NCT) expressed as a function of the temperature t, and

ISI(t) = International Sensitivity Index (ISI) expressed as a function of the temperature t.

It should be understood that the use of the term "vessel" comprises all kinds of containers for liquids, such as test tubes, cuvettes, vials etc.

In an embodiment of the invention, the liquid reagent comprises two or three different reagents.

In another embodiment the liquid reagent contains an amount of fibrinogen increasing the concentration of fibrinogen in the mixture of a) by at least 0.5 g/L and the ratio between the defined volume of the reagent and sample in a) is at least ten.

In a variant of the method of the invention, the measurement of the temperature in step b) is accomplished by determining the ambient room temperature, provided that the liquid reagent is of the same temperature.

As mentioned, the method of the invention may be performed at an ambient temperature of 18°C to 35°C. A most preferred temperature range under the conditions used in the examples is 30°C to 35°C.

In still another embodiment of the invention the prothrom bin time (PT) is obtained from a table with rows and columns where one is for various clotting times, and the other is various temperatures, and the PT is found in the intersection of clotting time and temperature.

A test kit for performing an analysis according to the method aspect of the invention comprises temperature recording means, and one or several separate sealed vessels, such as test tubes, cuvettes, vials etc., containing reagents for clotting one or more defined volumes whole blood, anti-coagulated blood, blood plasma or anti-coagulated blood plasma sample.

The temperature recording means can be any means with which the ambient temperature in the range of recording 15°C to 45°C can be recorded, such as an ordinary glass thermometer, a thermistor etc. The thermometer may also be an especially designed thermometer that has a hook at one end, thus enabling visual detection of clot formation in the vessel as the thermometer is moved to and fro.

A reagent in a vessel contains fibrinogen in an amount that yields a final fibrinogen concentration of at least 0.1 g/L in a vessel when mixed with a sample to be tested.

The reagents in the vessels may be in lyophilized form for reconstitution with one or more defined volumes of liquid prior to use.

Even though it is not necessary to provide time registration means, such as a stop watch or electronic timer with a test kit, it will be convenient for the end user to have all necessary equipment in one test kit. Therefore, it will also be suitable to include volume determining means, such as pipettes or the like into the test kit.

Calibration of a PT test involves the whole procedure or method. It involves the reagent(s), the instrument, the volumetric equipment and the instructions for use. Since many users, especially POC PT tests users, want to rely on the calibration of the manufacturer, it follows, that the calibration of a manufacturer will be reliable only if the product delivered, the equipment kit, contains everything needed to perform the PT test. However, according to prior art, this is not the case. Because of the high costs of the instrument, the instrument will be delivered separately and will be used throughout an extended time period, e.g. three to five years. The instrument will not be replaced every time a new lot of reagent is taken into use, which may be about every 6 to 12 months. However, the present invention will be useful in the development of a cheap instrument that can be disposed of after use of a reagent lot comprised by a test kit.

The main reason to perform a coagulation tests at ambient temperature is to abolish the need of expensive and energy consuming temperature control equipment. According to the present invention, it is possible to perform coagulation tests at the ambient temperatures typical of human dwellings. According to the present invention, it is possible to perform a coagulation test at ambient temperature in which the clotting time and the temperature are measured and used to obtain the test results. In performing a coagulation test according to the invention, there is also a second reason to measure the temperature. This is to qualify the temperature conditions of the test.

The test results of a PT test is expressed in INR. According to prior art, a PT test is performed at a fixed temperature of 37°C and the INR is a function of the clotting time, INR(CT). As opposed to this, a PT test is performed according to the invention at any temperature within the specified limits. The clotting time and the temperature (t) are both measured and the INR is a function of both, INR(CT, t). The temperature is also measured to ascertain that the temperature is within a range where the PT test according to the invention may be performed.

The coagulation test is intended to be performed at temperatures typical of human dwellings. Hence, the ambient temperature range is assumed to be the indoor temperature range of such a dwellings, i.e. between 15°C and 45°C. It is assumed within the scope of the present invention that the ambient temperature is limited to the range of 18°C and 35°C.

A sharp dependency of the clotting time with temperature is likely to reduce the precision of the assay. As evidenced in Example 1, the speed of clot formation is reduced at temperatures below 18°C making clotting less distinct and more difficult to detect. Also at temperatures above 35°C the PT results were dependent on temperature. It is therefore preferred in the present invention to perform PT test by a procedure at an ambient temperature in the range of 18°C to 35°C.

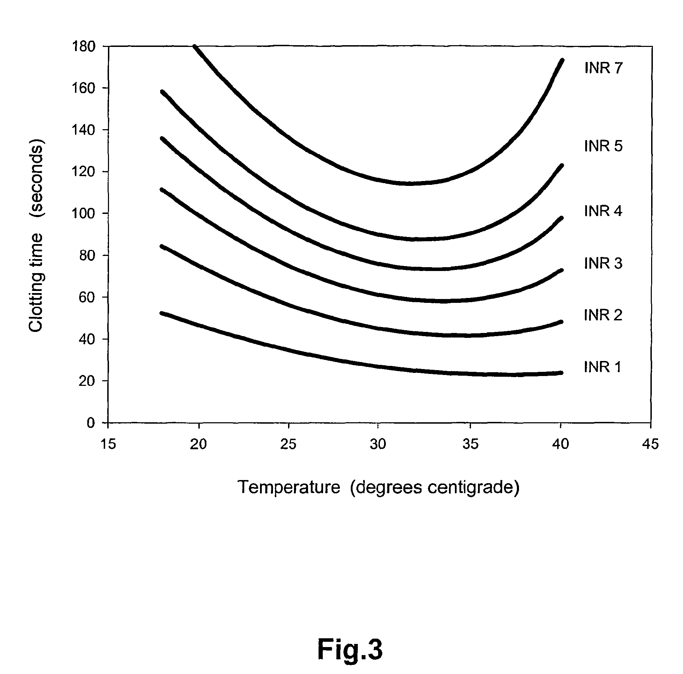

In antithrombotic treatment with vitamin K antagonists the optimal blood PT level is in the range of INR 2 to INR 3. The results of experimental work described in Example 1 indicate that there is an optimal temperature range for determination of PT in samples with INR above 2, see Figure 3. This optimal temperature range is about 30°C to 35°C. In performing coagulation tests according to the invention, it may be favorable to so in the ambient temperature range of 30°C to 35°C,

Other temperature limits than those mentioned above are possible. As indicated, the lower limit of the range of permissible temperatures appears to be the most critical and may needed to be fine tuned, e.g. specified to 22°C. Although, no reason has appeared why the upper limit of permissible temperatures should be lower than 35°C, such reason could well appear. Reduction of the upper limit, e.g. to 30°C, is possible within the scope of the present invention.

As mentioned above, there appears to be an optimal temperature range for determination of PT in samples with INR in the range 2 to 3 or even the range of 2 to 7. In this optimal temperature range, the temperature dependence of the test result is small which will minimize the impact of bad temperature equilibration and/or determination. In the examples below, this optimal temperature range was about 30°C to 35°C. The important observation is that there is an optimal temperature range and it is conceived that alterations in reagent composition and/or procedural details can shift this optimal temperature range to lower temperatures including those typically found in human dwellings, i.e. 20°C to 30°C. If such a shift in optimal temperature can be accomplished, then there are reasons to adjust the limits of permissible temperature accordingly.

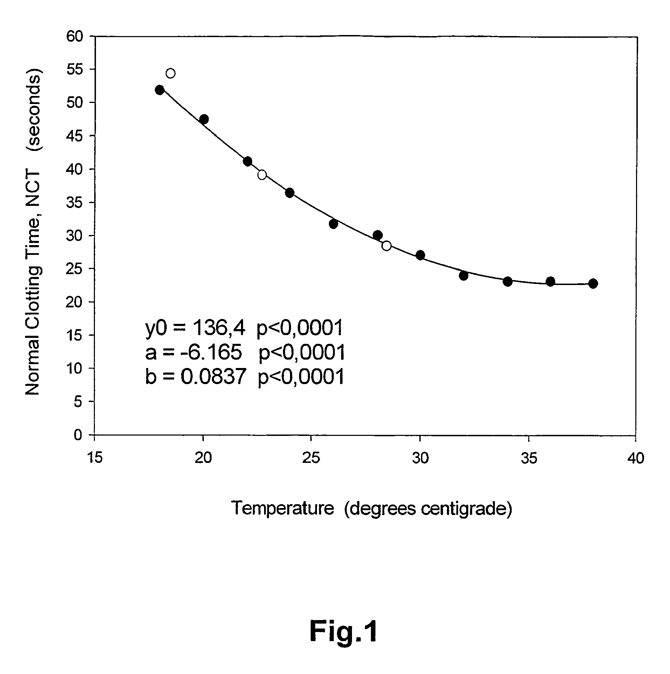

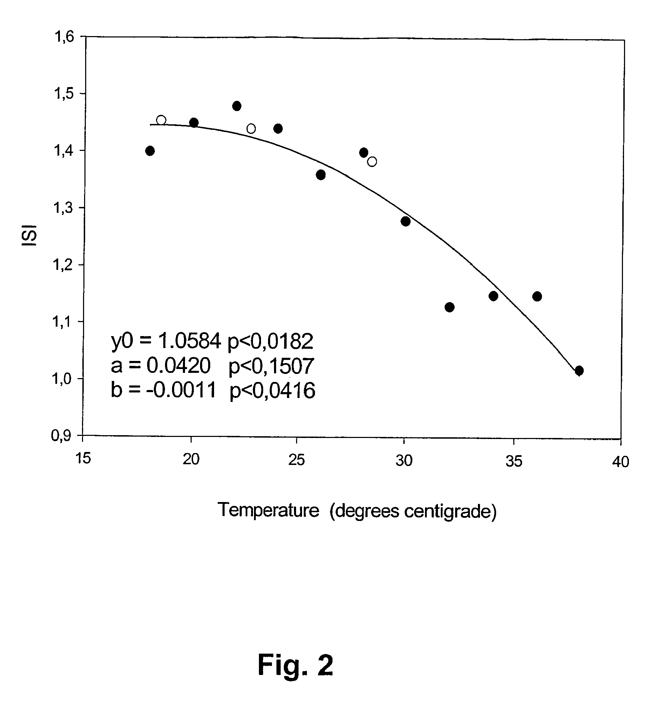

The invention also concerns how to obtain results from PT tests performed according to the invention. In a PT procedure according to the invention, the clotting time and the temperature are determined. As shown in Figure 1 and Figure 2, the NCT and the ISI for a given PT procedure can be viewed as functions of temperature and the nature of these functions may be determined by calibration. The PT procedure according to the invention can be calibrated by determining the clotting time at various temperatures within the permissible range for samples with known INR. At these temperatures, the NCT and the ISI of the PT procedure are determined and functional relationships between normal NCT and temperature and ISI and temperature. The later are denoted NCT(t) and ISI(t), respectively. Once, NCT(t) and ISI(t) are determined, the INR as function of clotting time (CT) and temperature, INR(CT, t) can be determined as follows:

$$INR(CT, t) = (CT/NCT(t))^{ISI(t)}$$

In the examples, NCT(t) and ISI(t) were second degree polynomials. Naturally many other types of functions may find use in this context. Any function that gives a good fit to the calibration data may be used.

As an alternative to deducing a functional relationship between INR and t and CT, i.e. deducing a two variable expression NCT(t, CT) and computing the assay results using this expression, a table may be constructed. This table will be a matrix where each row is a clotting time (CT) and each column a temperature. Each given pair of temperature and CT will correspond to an INR. Thus, there is no departing from the invention if the results of the PT procedure is presented in a table where the results of the test is in position defined by a temperature and a clotting time.

Any method according to claim 1 that allows an INR value to be deduced from a CT and a temperature may be employed. The same is true for assay conditions. These may include any limited permissible temperature range according to claim 1 and any limited permissible clotting time range. In the experimental work there are temperature ranges identified for which the temperature dependence is negligible and which makes the INR value a function of the clotting time alone. Also in this case the PT test is enabled by the invention. The clotting time and the temperature are both measured and used to obtain the test results. The temperature is measured to ascertain that the temperature is within a permissible range.

It is enabled by the invention to provide users equipment needed to perform a PT procedure according to the invention. A test kit may, in addition to the components already mentioned, contain an instrument and instructions for use. A test kit may e.g. contain reagents for 10 to 100 PT analysis.

**Description of the drawings**

Fig.1 is a diagram wherein the filled circles show the NCT of Table 1. The solid line is second-degree poly nomial fitted to the experimental data points by minimal least square method. The open circles show the NCT values for the PT procedure estimated with the patient plasma samples and the INR values for these supplied by the department. The fitted function was NCT(t) = 136.4 - 6.165t + 0.0837t$^2$.

Fig. 2 is a diagram wherein the filled circles show the experimentally determined ISI data of Table 1. The solid line is second-degree polynomial fitted to the experimental data points by minimal least square method. The open circles show the NCT values for the PT procedure estimated with the patient plasma samples and the INR values for these supplied by the department. The fitted function was ISI(T) = 1.0584 + 0.04201t - 0.0011t$^2$.

Fig. 3 is a diagram wherein the solid lines show the expected clotting time with the PT procedure for samples with INR values of 1, 2, 3, 4, 5 and 7 in the temperature range of 18°C to 40°C. The calculations were made with the relationship INR = (CT/NCT)$^{ISI}$. rearranged into INR = (CT/NCT(t))$^{ISI(t)}$, with insertions of the NCT(t) and ISI(t) given in legends to Figures 1 and 2.

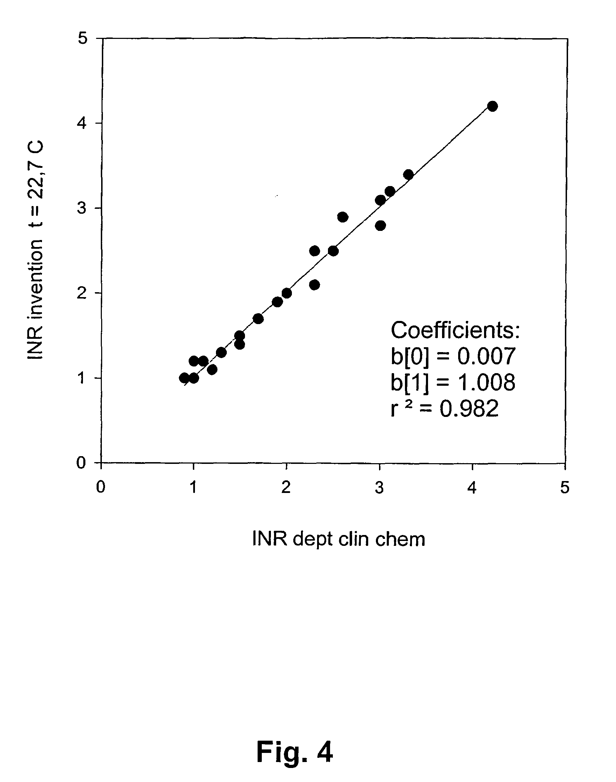

Fig. 4 is a diagram wherein INR values of 23 patient citrated plasma samples determined according to the invention at 22.7°C are compared with INR values for the same samples determined according to prior art. by the Department of Clinical Chemistry at the University Hospital, Linköping, Sweden.

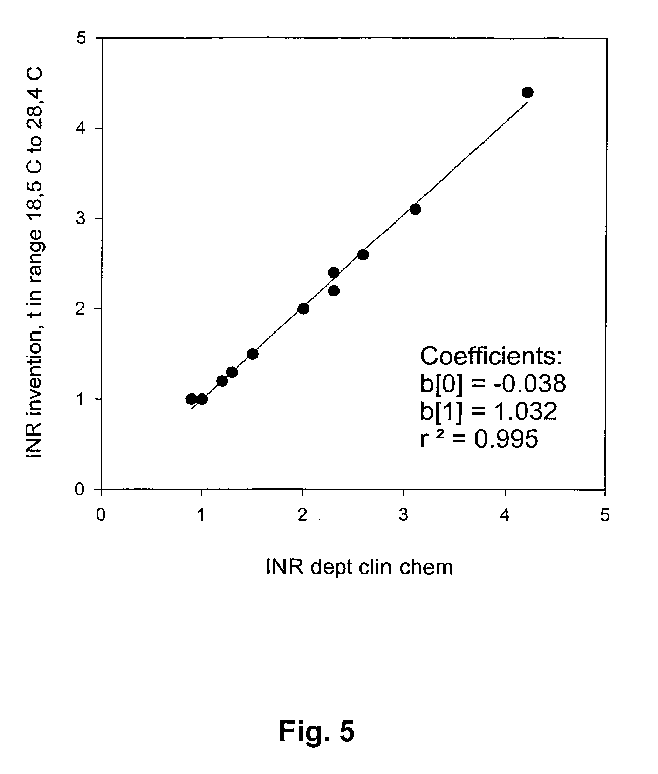

Fig. 5 is a diagram wherein the average INR value of three determinations according to the invention for 12 patient plasma samples, at 18.5°C, 22.7°C and 28.4°C, were compared to the INR values of the same samples determined according to prior art. The determinations according to the invention and according to prior art are denoted INR invention and INR dept clin chem, respectively.

## EXAMPLES

The following examples are given to a better understanding of the invention and to show that the invention is of practical and economic importance. The examples show but a trifle of all possible embodiments of the invention. Therefore, the examples do not limit in any way the present invention.

**Materials and Methods.**

PT reagent was GHI 131 lot C225F which is of the Owren's kind in that it contains rabbit brain thromboplastin and bovine factor V and bovine fibrinogen. The reagent, supplied as a lyophilized powder. One vial of reagent was reconstituted in first 5 mL of water and then 5 mL of 25 mM $CaCl_2$. The reagent was used within 4 to 10 hours of reconstitution. In the PT test procedure, 300 $\mu$L of reagent was placed in the reaction vessel to which 25 $\mu$L of sample was added, a ratio of 12. The amount of fibrinogen in the reagent was sufficient to increase the fibrinogen concentration in the mixture of sample and reagent by about 0.8 g/L.

Control plasma samples with known INR were lyophilized plasma samples with known INR. One control plasma, product number GHI 162, had an INR of 1.14 and the other, product number GHI 167, had an INR of 2.82. The lyophilized control plasmas were first reconstituted in 1 mL of water according to the manufacturers recommendation, and then, to give the reconstituted control plasma about the same PT potency as blood with erythrocyte fraction volume (EVF) of 0.45, the control plasma was diluted by addition of 0.82 mL of 150 mM NaCl. The PT reagent and control plasmas were manufactured by Global Hemostasis Institutet MGR AB, Linköping, Sweden.

Citrated plasma samples were from the Department of Clinical Chemistry, University Hospital, Linköping, Sweden. The 23 samples were from those arriving to the department for analysis of PT during about 3 hours in the afternoon of June 12, 2003. The samples were analyzed according to the invention within 4 hours of being analyzed by the routine procedure of the department. The samples were made untraceable to preserve the anonymity of the patients and were provided with the PT values obtained by the department. Prior to assay according to the invention, a 200 $\mu$L portion of each plasma was diluted 1:1.82 by addition of 164 $\mu$L of 150 mM NaCl. This was to make them equipotent with blood with EVF of 0.45. The clinical experiments were conducted in collaboration with Professor Tomas L. Lindahl, Department of Clinical Chemistry, University of Linköping and approved by the local ethical committee of the Medical Faculty of the University of Linköping.

Glass capillaries with a volume of 25 $\mu$L (length 50 mm), product number 111.395-25, were from KEBO-lab, Stockholm., Sweden.

The PT reagent was added to 8 mm inner diameter polystyrene tubes, 300 $\mu$L of reagent in each. The tubes were capped and temperature equilibrated in a water bath. At time zero, 25 $\mu$L of sample was added to a reagent tube and recapped. The tube was tilted once about every second so that the bottom of the tube was out of water bath when tilted and back in the water bath when straight up. The contents were inspected every time the tube was tilted. When first signs of clotting were detected the time was recorded. The temperature in the water bath was varied between 14°C and 40°C. The 25 $\mu$L of sample was added either by glass capillary or by pipette with disposable plastic tip.

**Example 1.**

With a PT procedure, the clotting time (CT) was determined for two control plasma samples at every two degrees centigrade in the temperature range of 18°C to 38°C. The control plasma samples had INR values of 1.14 and 2.82. From these values, the Normal Clotting Time (NCT) and International Sensitivity Index (ISI) of the PT procedure were calculated at each of the eleven temperatures. The NCT was in the range of 55 sec to 22 sec and the ISI was in the range 1.0 to 1.5, See Figures 1 and 2. Second-degree polynomials were fitted by minimal least square method to give NCT and ISI as functions of temperature. The second-degree polynomials that gave the best fit to the data were:

$$NCT(t) = 136.4 - 6.165t + 0.0837t^2$$

$$ISI(t) = 1.0584 + 0.0420t - 0.0011t^2$$

NCT and ISI for the same PT procedure was also determined at three temperatures, 18.5°C, 22.7°C and 28.4°C with patient plasma samples from the Department of Clinical Chemistry. Figure 1 and 2 also show that the estimates of NCT and ISI made with the patient plasma samples and the INR values supplied by the department were well in line.

The functional relationship between the temperature and NCT and ISI allowed the construction of schools of curves, each for a specific INR value, showing the expected clotting time as function of temperature. The curve for INR 1 flattened out in the temperature range of 35°C to 40°C. The curves for higher INR values displayed an increasingly pronounced minimum in the range 30°C to 35°C.

Example 1 demonstrates that functional relationships of temperature and NCT and ISI, NCT(t) and ISI(t), can be determined. Furthermore, Example 1 demonstrates that every value pair (t, CT) translates into an INR value. Figure 3 could be used for such a translation but an interpolation between the curves is necessary. A two variable mathematical relationship or a table appears to be the most straight forward means to translate the (t, CT) value pair into an INR value. Figure 3 clearly demonstrates that a translation of this kind can be performed in practice.

When a sample is analyzed according to the invention, the clotting time and the ambient temperature (t) are measured. With these values and known functional relationships NCT (t) and ISI(t), an INR value can be computed as $INR = (CT/NCT(t))^{ISI(t)}$. Thus, according to the invention, provided the temperature is within a permissible range, every value pair (t, CT) will generate an INR value. Naturally, the CT must also be within some permissible range but this does not represent anything new in comparison to state of art PT procedures and is therefore not elaborated on here.

**Example 2.**

Plasma samples from 23 patients were obtained from the Department of Clinical Chemistry together with INR values determined according to prior art. The 23 samples were analyzed according to the invention. The ambient temperature and the clotting time for each sample were measured. The temperature in the series was stable at 22.7°C. At this temperature, the NCT and ISI of the PT procedure was determined with respect to the prior art INR values. INR values according to the invention were then computed. The INR values obtained according to the invention at 22.7°C were compared to the INR values obtained according to prior art at 37°C, see Figure 4. The comparison was by linear regression. A slope of near unity (1.008), an intercept close to zero (0.007) and a correlation coefficient of 0.982 was obtained. There were no obvious outliers. The first 12 of the 23 samples were similarly analyzed at 18.5°C and 28.4°C. For each of these 12 samples a mean INR value was determined as the average of three determinations, one at each of the three mentioned temperatures. This mean INR value was also compared by linear regression analysis to the INR values supplied by the department, Figure 5. Interestingly, the mean INR values correlate more strongly with the INR values supplied by the department than the INR values obtained at only one temperature (22.7°C), squared regression coefficient was 0.995 compared to 0.982. Inspection of the data in Figure 5, shows no bias and no obvious outliers. It appears that INR can be determined according to the invention at any temperature in the range of 18°C to 40°C.

Example 2 demonstrates that INR values can be determined at other temperatures than 37°C. According to the data, a PT test can be performed at ambient temperature provided that the clotting time and the temperature are measured and that both of these values are used to derive the test result. To further prove the point, mean INR values were determined for 12 patient plasma samples. Each mean INR value was an arithmetic average of three INR determinations, one at 18.5°C, a second at 22.7°C and a third at 28.4°C. The mean INR values and the prior art INR values were compared in Figure 5. In this case the data lined up even better that in Figure 4. The results are interpreted in the following way; each mean INR value is the average three determinations according to the invention. Each of these three estimates is equally good, regardless of temperature. The mean INR values are therefore better estimates of the true INR value than the single estimate at 22.7°C. A standard deviation of the determinations are obtained by assuming that the INR value of the department was correct. The standard deviation for the mean INR and the 22.7°C INR can then be determined to 0,029 and 0.043, respectively. The ratio between these standard deviations is 1.5 which is close to 1.73, the square root of three, which is expected if all determinations, regardless of temperature, carry the same weight.

The data shown in Figures 4 and 5 display a discovery on which the invention is based. PT can be determined at any temperature in the range of about 15°C to 45°C. The temperature needs only be measured and used together with the clotting time to obtain the assay result. The fact that PT can be determined over a relatively broad temperature range is not trivial. It is a fact that now has been experimentally verified. Now we know, that new analytical possibilities, offering considerable advantages, open themselves unto us. Since the temperature does not by necessity need to be defined, it only needs to be measured, much expensive hardware can be stripped from the instrument used for PT determination. It could well be that the instruments can perhaps be made so inexpensive that a disposable instrument for PT tests is possible. For POC PT testing this could be decisive.

It is likely that the invention may be practiced on any type of wet-chemistry PT test. In particular is clearly demonstrated that the invention may be practiced with a wet-chemistry PT test of the Owren type, i.e. with PT test in which the reagent contains sufficient amounts of fibrinogen to increase the fibrinogen concentration in the mixture of at least in the range of 0.1 g/L to 0.5 g/L and in which the volumetric ratio reagent to sample is at or above the range of four to ten. The volumetric ratio may, however, be much higher, e.g. 50 or even higher.

PT determination according to the invention could result in improved test quality. One reason for a bad PT result is temperature fault. The temperature in the reaction mixture is not what it is assumed to be. This could be because of the temperature has not had the time to equilibrate, or because of some error in the thermostat. It stands clear to reason that it is easier to measure a temperature with good precision than to keep it at some predetermined level with the same good precision.

**Table 1**. The PT clotting time for the two control plasma samples were determined at various temperatures in the range of 18°C to 38°C. The clotting times are designated CT INR 1.14 and CT INR 2.82. Using these two CT values, the NCT and the ISI of the PT procedure was determined at each temperature from the relationship INR = $(CT/NCT)^{ISI}$. All clotting times are given in seconds.

| Temperature | CT INR 1.14 | CT INR 2.82 | NCT | ISI |
|---|---|---|---|---|
| 18°C | 57 | 109 | 51.9 | 1.40 |
| 20°C | 52 | 97 | 47.5 | 1.45 |
| 22°C | 45 | 83 | 41.2 | 1.48 |
| 24°C | 40 | 75 | 36.5 | 1.44 |
| 26°C | 35 | 68 | 31.8 | 1.36 |
| 28°C | 33 | 63 | 30.1 | 1.40 |
| 30°C | 30 | 61 | 27.1 | 1.28 |
| 32°C | 27 | 60 | 24.1 | 1.13 |
| 34°C | 26 | 57 | 23.2 | 1.15 |
| 36°C | 26 | 57 | 23.2 | 1.15 |
| 38°C | 26 | 63 | 22.9 | 1.02 |

**Table 2**. Below is a piece of an Excel file showing that a clotting time and a temperature, measured when practicing a PT procedure according to the invention, can be translated into an INR value. There is one row for each CT in seconds and one column for each temperature in degrees centigrade. If in practicing a PT procedure according to the invention, a temperature of 24°C and a clotting time of 45 seconds are measured, then the INR of the sample is 1.34. The section of a matrix shown covers the temperature range 19°C to 28°C. If, in connection with a PT test performed according to the invention, a temperature lower than 18°C is determined, then this is outside the conditions under which the test is intended, and no INR value is obtained. The temperature measured is thus used to qualify test conditions. If the measured temperature is outside the permissible range, then the test conditions are not qualified and no test result will be generated.

| | | Temperature (°C) | | | | | | | | | |
|---|---|---|---|---|---|---|---|---|---|---|---|
| | | 19 | 20 | 21 | 22 | 23 | 24 | 25 | 26 | 27 | 28 |
| CT: | 29 | 0.46 | 0.50 | 0.55 | 0.60 | 0.66 | 0.71 | 0.78 | 0.84 | 0.91 | 0.98 |
| | 30 | 0.48 | 0.53 | 0.58 | 0.63 | 0.69 | 0.75 | 0.82 | 0.89 | 0.96 | 1.03 |
| | 31 | 0.51 | 0.55 | 0.60 | 0.66 | 0.72 | 0.79 | 0.86 | 0.93 | 1.00 | 1.08 |
| | 32 | 0.53 | 0.58 | 0.63 | 0.69 | 0.76 | 0.82 | 0.90 | 0.97 | 1.05 | 1.12 |
| | 33 | 0.55 | 0.60 | 0.66 | 0.72 | 0.79 | 0.86 | 0.94 | 1.01 | 1.09 | 1.17 |
| | 34 | 0.58 | 0.63 | 0.69 | 0.75 | 0.82 | 0.90 | 0.98 | 1.06 | 1.14 | 1.22 |
| | 35 | 0.60 | 0.66 | 0.72 | 0.79 | 0.86 | 0.94 | 1.02 | 1.10 | 1.19 | 1.27 |
| | 36 | 0.63 | 0.69 | 0.75 | 0.82 | 0.89 | 0.97 | 1.06 | 1.15 | 1.23 | 1.32 |
| | 37 | 0.65 | 0.71 | 0.78 | 0.85 | 0.93 | 1.01 | 1.10 | 1.19 | 1.28 | 1.37 |
| | 38 | 0.68 | 0.74 | 0.81 | 0.89 | 0.97 | 1.05 | 1.14 | 1.24 | 1.33 | 1.42 |
| | 39 | 0.71 | 0.77 | 0.84 | 0.92 | 1.00 | 1.09 | 1.19 | 1.28 | 1.38 | 1.47 |
| | 40 | 0.73 | 0.80 | 0.87 | 0.96 | 1.04 | 1.13 | 1.23 | 1.33 | 1.43 | 1.53 |
| | 41 | 0.76 | 0.83 | 0.91 | 0.99 | 1.08 | 1.17 | 1.27 | 1.38 | 1.48 | 1.58 |
| | 42 | 0.79 | 0.86 | 0.94 | 1.03 | 1.12 | 1.22 | 1.32 | 1.42 | 1.53 | 1.63 |
| | 43 | 0.81 | 0.89 | 0.97 | 1.06 | 1.16 | 1.26 | 1.36 | 1.47 | 1.58 | 1.68 |
| | 44 | 0.84 | 0.92 | 1.01 | 1.10 | 1.20 | 1.30 | 1.41 | 1.52 | 1.63 | 1.74 |
| | 45 | 0.87 | 0.95 | 1.04 | 1.13 | 1.23 | 1.34 | 1.45 | 1.57 | 1.68 | 1.79 |
| | 46 | 0.90 | 0.98 | 1.07 | 1.17 | 1.27 | 1.38 | 1.50 | 1.62 | 1.73 | 1.85 |
| | 47 | 0.93 | 1.01 | 1.11 | 1.21 | 1.31 | 1.43 | 1.55 | 1.67 | 1.79 | 1.90 |

(continued)

| | Temperature (°C) | | | | | | | | | |
|---|---|---|---|---|---|---|---|---|---|---|
| | 19 | 20 | 21 | 22 | 23 | 24 | 25 | 26 | 27 | 28 |
| 48 | 0.96 | 1.04 | 1.14 | 1.24 | 1.36 | 1.47 | 1.59 | 1.72 | 1.84 | 1.96 |
| 49 | 0.99 | 1.08 | 1.18 | 1.28 | 1.40 | 1.52 | 1.64 | 1.77 | 1.89 | 2.02 |
| 50 | 1.02 | 1.11 | 1.21 | 1.32 | 1.44 | 1.56 | 1.69 | 1.82 | 1.95 | 2.07 |

**Claims**

1. A method of determining, in a coagulation test, prothrombin time (PT) in a whole blood, anti-coagulated blood, blood plasma or anti-coagulated blood plasma sample comprising the steps of

a) adding a defined volume of the sample to a defined volume of a liquid reagent comprising thromboplastin and fibrinogen in a ratio between the defined volumes of the reagent and the sample of at least four, the amount of fibrinogen increasing the concentration of fibrinogen in the mixture by at least 0.1 g/L, or vice versa, in a vessel,
b) measuring the temperature of the reaction mixture of a) having an ambient temperature in the range of 18°C to 35°C,
the steps a) and b) being performed in an optional order, followed by
c) determining a clotting time (CT) by registering the time from the addition in a) until a clot formation is detected in the vessel,
and
d) calculating the prothrombin time (PT) based on the temperature of b) and the clotting time (CT) of c), expressed as International Normalized Ratio (INR), by using the equation.

$$INR = (CT/(NCT(t))^{ISI(t)}$$

wherein
CT the clotting time of c) in claim 1,
t = the temperature of b) in claim 1,
NCT(t) = Normal Clotting Time (NCT) expressed as a function of the temperature t, and
ISI(t) = International Sensitivity Index (ISI) expressed as a function of the temperature t.

2. The method according to claim 1, wherein the liquid reagent comprises two or three different reagents.

3. The method according to claim 1 or 2, wherein the liquid reagent contains an amount of fibrinogen increasing the concentration of fibrinogen in the mixture of a) by at least 0.5 g/L and the ratio between the defined volume of the reagent and sample in a) is at least ten.

4. The method according to any one of claims 1-3, wherein the measurement of the temperature in b) is accomplished by measuring the ambient room temperature, provided that the liquid reagent is of the same temperature.

5. The method according to claim 1, wherein the ambient temperature is in the range of 30°C to 35°C.

6. The method according to any one of claims 1 to 5. wherein the prothrombin time (PT) is obtained from a table with rows and columns where one is for various clotting times, and the other is various temperatures, and the PT is found in the intersection of clotting time and temperature.

**Patentansprüche**

1. Verfahren zum Bestimmen der Prothrombinzeit (PT) in einer Vollblut-, antikoagulierten Blut-, Blutplasma- oder antikoagulierten Blutplasmaprobe in einem Koagulationstest, folgende Schritte umfassend:

a) Zugeben eines definierten Volumens der Probe zu einem definierten Volumen eines flüssigen thromboplastin- und fibrinogenhaltigen Reagens in einem Verhältnis zwischen den definierten Volumina des Reagens und der Probe von mindestens vier, wobei die Fibrinogenmenge den Fibrinogengehalt im Gemisch um mindestens 0,1 g/L erhöht, oder umgekehrt, in einer Küvette,

b) Messen der Temperatur des Reaktionsgemischs von a), das eine Umgebungstemperatur im Bereich von 18 °C bis 35 °C aufweist,

wobei die Schritte a) und b) in beliebiger Reihenfolge durchgeführt werden, gefolgt von

c) Bestimmen einer Gerinnungszeit (CT) durch Erfassen der Zeit ab dem Zugeben in a) bis eine Gerinnselbildung in der Küvette festgestellt wird,

und

d) Berechnen der Prothrombinzeit (PT) basierend auf der Temperatur von b) und der Gerinnungszeit (CT) von c), ausgedrückt als International Normalized Ratio (INR), anhand der Gleichung

$$INR = (CT/(NCT(t))^{ISI(t)}$$

wobei

CT = die Gerinnungszeit von c) in Anspruch 1,

t = die Temperatur von b) in Anspruch 1,

NCT(t) = Normale Gerinnungszeit (NCT), ausgedrückt als Funktion der Temperatur t, und

ISI(t) = Internationaler Empfindlichkeitsindex (ISI), ausgedrückt als Funktion der Temperatur t.

2. Verfahren nach Anspruch 1, wobei das flüssige Reagens zwei oder drei verschiedene Reagenzien umfasst.

3. Verfahren nach Anspruch 1 oder 2, wobei das flüssige Reagens eine Fibrinogenmenge enthält, die den Fibrinogengehalt im Gemisch von a) um mindestens 0,5 g/L erhöht, und das Verhältnis zwischen dem definierten Volumen des Reagens und der Probe in a) mindestens zehn beträgt.

4. Verfahren nach einem der Ansprüche 1 bis 3, wobei das Messen der Temperatur in b) durch Messen der Raum-Umgebungstemperatur erfolgt, sofern das flüssige Reagens die gleiche Temperatur aufweist.

5. Verfahren nach Anspruch 1, wobei die Umgebungstemperatur im Bereich von 30 °C bis 35 °C liegt.

6. Verfahren nach einem der Ansprüche 1 bis 5, wobei die Prothrombinzeit (PT) aus einer Tabelle mit Zeilen und Spalten für unterschiedliche Gerinnungszeiten bzw. unterschiedliche Temperaturen hervorgeht und die PT am Kreuzungspunkt der Gerinnungszeit und der Temperatur steht.

**Revendications**

1. Procédé permettant de déterminer, dans un test de coagulation, le temps de prothrombine (PT) dans un échantillon de sang total, de sang anticoagulé, de plasma sanguin ou de plasma sanguin anticoagulé, comprenant les étapes consistant à

a) ajouter un volume défini de l'échantillon à un volume défini d'un réactif liquide contenant de la thromboplastine et du fibrinogène à un rapport entre les volumes définis du réactif et de l'échantillon d'au moins quatre, la quantité de fibrinogène augmentant la teneur en fibrinogène dans le mélange d'au moins 0,1 g/L, ou vice-versa, dans une cuvette,

b) mesurer la température du mélange réactionnel de a) ayant une température ambiante dans le domaine de 18 °C à 35 °C,

les étapes a) et b) étant effectuées dans un ordre arbitraire et suivies des étapes consistant à

c) déterminer un temps de coagulation (CT) en enregistrant le temps à partir de l'ajout en a) jusqu'à ce que la formation d'un caillot soit détectée dans la cuvette,

et d) calculer le temps de prothrombine (PT) sur la base de la température de b) et le temps de coagulation (CT) de c), exprimé sous la forme du rapport international normalisé (RIN), en utilisant l'équation

$$RIN = (CT/(NCT(t))^{ISI(t)}$$

où
CT = le temps de coagulation de c) à la revendication 1,
t = la température de b) à la revendication 1,
NCT(t) = temps normal de coagulation (NCT) exprimé en fonction de la température t, et
ISI(t) = indice de sensibilité international (ISI) exprimé en fonction de la température t.

2. Procédé selon la revendication 1, où le réactif liquide comprend deux ou trois réactifs différents.

3. Procédé selon la revendication 1 ou 2, où le réactif liquide contient une quantité de fibrinogène qui augmente la teneur en fibrinogène dans le mélange de a) d'au moins 0,5 g/L et le rapport entre le volume défini du réactif et de l'échantillon en a) est d'au moins dix.

4. Procédé selon l'une quelconque des revendications 1 à 3, où la mesure de la température en b) est réalisée en mesurant la température ambiante de laboratoire, tant que le réactif liquide présente la même température.

5. Procédé selon la revendication 1, où la température ambiante est dans le domaine de 30 °C à 35 °C.

6. Procédé selon l'une quelconque des revendications 1 à 5, où le temps de prothrombine (PT) apparaît sur une table ayant des lignes et des colonnes où l'une représente différents temps de coagulation et l'autre représente différentes températures et le PT se trouve à l'intersection du temps de coagulation et de la température.

y0 = 136,4  p<0,0001
a = -6.165  p<0,0001
b = 0.0837  p<0,0001

**Fig.1**

y0 = 1.0584  p<0,0182
a = 0.0420   p<0,1507
b = -0.0011  p<0,0416

**Fig. 2**

Fig.3

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6402704 B1, McMorrow
- US 6103196 A, Yassinzadeh
- US 5302348 A, Cusack
- US 4849340 A, Oberhardt
- US 3951606 A, Moyer
- US 6338821 B, Jina
- WO 9322453 A

**Non-patent literature cited in the description**

- *J Biol Chem,* 1935, vol. 109, 73-4
- **OWREN P ; AAS K.** The control of dicumarol therapy and quantitative determination of prothrombin and proconvertin. *Scand J Clin Lab Invest,* 1951, vol. 1951, 201-8
- *Thromb Haemost,* 2004, vol. 91, 1223-31
- **VAN DEN BESSELAAR AM.** International normalized ratio: towards improved accuracy. *Thromb Haemost,* 1999, vol. 82, 1562-3
- **POLLAR L et al.** Concerted Action on Anticoagulation (ECAA) - the multicentre calibration of rabbit and human ECAA reference thromboplastins. Steering Committee. *Thromb Haemost,* 1996, vol. 76, 977-82